# EUROPEAN PATENT SPECIFICATION

(11) **EP 1 871 321 B1**
(45) Date of publication and mention of the grant of the patent: **15.10.2008**
(21) Application number: 06738234.1
(22) Date of filing: 14.03.2006
(51) Int. Cl.: A61F 13/56, A61F 13/62, A44B 18/00

(54) **ABSORBENT ARTICLE**
SAUGFÄHIGER ARTIKEL
ARTICLE ABSORBANT

(30) Priority: 18.03.2005 EP 05006036
(43) Date of publication of application: 02.01.2008
(73) Proprietor: 3M Innovative Properties Company, St. Paul MN 55133-3427 (US)
(72) Inventor: PETERSEN, Johann, F., 41453 Neuss (DE); OERTEL, Ralf, G., 41453 Neuss (DE)
(74) Representative: Wilhelm, Stefan
(86) International application number: PCT/US2006/009152
(87) International publication number: WO 2006/101844

(56) References cited:
- EP-A- 0 988 808
- EP-A- 1 269 958
- WO-A-01/43684
- US-A- 5 300 058
- US-A- 5 933 927

## Description

### Technical Field

The present invention relates to an absorbent article for positioning in an undergarment to absorb body exudates and to a method of manufacturing such absorbent articles. Generally, these kinds of absorbent articles are known, e.g., as feminine hygiene or femcare products, in particular sanitary napkins, or adult incontinence products.

### Background Art

EP 0 988 808 A1 discloses a hook fastening material for mechanical fastening systems of the hook and loop type. This hook fastening material comprises different prongs to allow for an effective engagement with a variety of various loop materials. The engaging means of the prongs may vary in shape, material composition or dimensions. The hook fastening material disclosed in EP 0 988 808 A1 may be used to affix a disposable absorbent article to an undergarment of a wearer.

WO 99/03369 discloses a finger grip for a mechanical fastener system, wherein the hook elements in the finger grip area are treated to become non-functional to allow for a convenient grip. This fastening system is used for disposable articles such as diapers, incontinence garments, and the like.

US 6 210 389 B1 also discloses a fastener system with a lift region. A first fastener component includes an engagement substrate having an appointed lift region, and a plurality of engagement members which are operably attached to extend away form the engagement substrate. The lift region is disposed along at least a longitudinally extending, outboard edge of the engagement substrate, and the lift region contains a plurality of engagement members which have been substantially deactivated.

WO 97/32556 relates to a mechanical fastening system with a grip tab. The fastening system includes a hook base layer which has an appointed fastening region and an appointed grip region. The fastening region has a plurality of hook elements which are integrally formed with the base layer and extend away from a base plane of the hook base layer. The hook elements are configured to operably engage a selected, cooperating loop material. The grip region has a relatively lower density of the hook elements per unit area, as compared to the fastening region.

US 6 276 032 B1 relates to a fastening system to be applied on a diaper where the fastening tab comprises stems and hooks. The height of the stems and hooks may vary in order to provide fastening systems having a desired combination of comfort, ease of fastening, strong securement and ease of unfastening. The article disclosed in US 6 276 032 B 1 comprises a fastening system of male and female fasteners.

US 5 300 058 A relates to a sanitary napkin comprising oriented hooks with a different orientation in different quadrants of the napkin. This construction is said to provide a sanitary napkin that can be more securely affixed to the undergarment of a wearer.

### Summary of the Invention

It is an object of the present invention to provide an absorbent article for positioning in an undergarment to absorb body exudates, wherein the article can be securely attached to the undergarment and minimizes potential damage to the undergarment due to the positioning means. This object is achieved with the absorbent article as defined in the claims.

According to the present invention, the absorbent article for positioning in an undergarment to absorb body exudates, e.g., a sanitary napkin, comprises a main portion having a liquid-permeable body side sheet, a liquid-impermeable garment side sheet opposite to the body side sheet, and a liquid-absorbent core between the body side sheet and the garment side sheet. The article has an outer edge defining the shape of the article and a center area. The liquid-impermeable garment side sheet comprises at least two regions of fastening elements with different engagement characteristics, i.e. engageability. A first region with higher engageability is located at the center area of the article, and at least one further region with lower engageability is located between the center area and the outer edge.

The center area of the absorbent article can generally be considered to be the area around the geometrical center of the article and does not necessarily extend to the center so that, e.g., a ring-shaped region around the center can also constitute the center area. The center area preferably covers at least 50 %, more preferably at least 60 %, most preferably at least 75 % of the total area of the article.

The engageability of the fastening elements with an undergarment may be determined and defined by using standard undergarment materials. Generally, the term "engageability" as used herein relates to the ability of the absorbent article to be mechanically attached to an undergarment, wherein a higher engageability particularly refers to higher peel strength, higher dynamic shear strength and/or higher dynamic friction.

Although the region(s) with lower engageability may be provided only at distinct sections between the center area and the edge of the article, it is preferred that the fastening elements with lower engageability are present at least at opposing transverse, i.e. short, edges of the article. The region(s) with lower engageability may, however, also be provided along the whole edge or contour line of the article, i.e. the further region(s) preferably extends completely along the circumference of the article.

According to a preferred embodiment at least two regions of fastening elements with different engageability are provided on the absorbent article, i.e. the first region and the at least one further region are provided with mechanical fastening elements which have different engageability. It is also within the scope of the present invention that the engageability characteristics of the fastening elements change gradually, e.g., from the edge towards the center area of the article so that the fastening elements have incrementally or continuously increasing engageability. In accordance with the invention it is also contemplated that the at least one further region comprises a region without mechanical fastening elements. Such a region may be provided, for example, at the transverse edges of the article.

The engageability of the fastening elements can be varied by various methods. For example, the fastening elements of the at least one further region may have lower average height than the fastening elements of the first region. Generally, the difference in height between the fastening elements of the at least one further region and the fastening elements of the first region may be at least 10 percent, preferably at least 20 percent, and more preferably at least 50 percent, based on the height of the fastening elements of the first region. For instance, the difference in height between the fastening elements of the at least one further region and the fastening elements of the first region may be at least 50 µm, preferably at least 80 µm. Preferably, the height of the fastening elements in the at least one further region may be in the range of between 30-400 µm, preferably 75-300 µm, more preferably 150-250 µm, and the height of the fastening elements in the first region may be in the range of between 50-500 µm, preferably 100-350 µm, more preferably 200-300 µm.

Alternatively, or in addition to varying heights of the fastening elements in the various regions, fastening elements of different types may be provided in the first and further regions. For example, the fastening elements of the first region may be provided in the form of hook-type or mushroom-type fastening elements and the fastening element of the at least one further region may be stems with smaller hook heads or mushroom heads or eventually without heads. It would also be possible to provide the fastening elements of the at least one further region in the form of mushroom-type hooks with a generally circular cap and in the first region mushroom-type hooks with a generally elliptical cap to increase their engageability. More generally, the size and/or shape of the heads of the male fastening elements may be varied so as to provide a suitable engageability.

Another possibility of varying the engageability characteristics of the fastening elements in the first and further regions is to alter the density of the fastening elements. For example, the fastening elements of the at least one further region may be provided with a lower density per unit area than the fastening elements of the first region. The density of the fastening elements may generally be in the range from about 30 to 2500 hooks per square centimeter. Preferably, the hook density is in the range from about 150 to 500 hooks per square centimeter, more preferably in the range from about 200 to 300 hooks per square centimeter.

Another option in order to vary the engageability of the fastening elements is to change the cross-sectional area of stems of the fastening elements. With stems of the fastening elements in the at least one further region having a smaller cross-sectional area than the fastening elements of the first region a lower engageability in the at least one further region than in the first region can be achieved. The material properties of the fastening elements may also be varied, e.g., more flexible material may be used for the fastening elements in the at least one further region and stiffer material in the first region. Combinations of the above-mentioned methods for varying the engageability are also contemplated.

Furthermore, the mechanical fastening elements in at least one of the first and further regions may be provided with an adhesive. Preferably, the adhesive is provided in a pattern of varying tackiness. For example, the hook and/or stem can be coated with an adhesive as taught, e.g., in US-A-6,489,003, US-A-6,428,525, US-A-6,402,730, US-A-6,393,673, and US-A-4,959,265. Alternatively and preferably, the adhesive may be provided between the stems and/or hooks as disclosed, e.g., in US-A-5,053,028 and US-A-5,019,065.

The absorbent articles of the present invention typically have an elongated shape, e.g. generally oval or elliptical, triangular, hourglass or keyhole shape. Advantageously, the main body of the article may comprise at each of its two opposite longitudinal edges flaps that are adapted to be folded over the opposite side of the undergarment. Each of these flaps may comprise a first region with fastening elements of higher engageability located at the center area of the flap and at least one further region with fastening elements of lower engageability located between the center area and the edge of the flap. Typically, the at least one further region of the flaps comprises the same or similar fastening elements as the at least one further region of the main body, and the first region of the flaps comprises the same or similar fastening elements as the first region of the main body.

Generally, the engageability of the fastening elements of the at least one further region with the undergarment may be at least 20 %, preferably at least 35 % and more preferably at least 50 % lower than the engageability of the fastening elements of the first region.

With the absorbent article according to the present invention, which has particularly at its edge areas a lower engageability, damage to the undergarment can be effectively minimized. It is believed that this positive effect is due to the lower engageability, particularly lower peel resistance, of at least one further region which is located between the center area and the edge of the absorbent article. While the peel resistance of the fastening elements in this at least one further region is rather small, the shear strength and friction of the fastening elements of the at least one further region are preferably still adequate and sufficient so as to assist in positioning and securely holding the absorbent article in the undergarment.

The invention also relates to a method of manufacturing an absorbent article for positioning in an undergarment to absorb body exudates. This method comprising the steps of providing a main portion having a liquid-permeable body side sheet, a liquid-impermeable garment side sheet opposite to said body side sheet, and a liquid-absorbent core between said body side sheet and said garment side sheet, and providing on said liquid-impermeable garment side sheet at least two regions of fastening elements with different engageability. A first region with higher engageability is located at a center area of the article, and at least one further region with lower engageability is located between the center area and an outer edge of the article.

The regions with fastening elements of different engageability may be provided particularly by joining or laminating a separate hook carrier web to a given garment side sheet of the sanitary napkin. This lamination can be done by the use of an adhesive, application of thermal and/or sonic means. Alternatively, these regions may be integrally provided on the napkin.

Preferably, the first region and the at least one further region are provided with mechanical fastening elements by means of a micro-replication process. For example, micro-replicated stems may be provided from a master belt, master drum, master screen, or any other form. The master belt, drum or screen is typically formed as a negative with differences in height, diameter, shape, density in registry to build the corresponding web with stems of the various regions. For instance, the master belt, drum or screen may be provided with openings or cavities by use of drilling, etching, laser erosion, punching or similar electrical, mechanical or chemical processing.

In accordance with a preferred method, the mechanical fastening elements are provided by first forming a plurality of stems on a substrate web, and then capping the stems in the first region so as to form mushroom-type mechanical fastening elements. The stems in the first region are preferably provided with a larger height than the stems in the at least one further region. It is preferred that at least some of the fastening elements of the at least one further region located generally at the edge of the article remain uncapped. Accordingly, the substrate web and the stems are produced from the same material. Alternatively, the substrate web may be different in chemistry to the stems. Suitable substrate webs may be paper, nonwoven, all film type constructions, scrim, etc.

Another manufacturing technique suitable for the absorbent article of the present invention is polymeric printing as disclosed, e.g., in US 2001/0018110 A1, US 2001/0016245 A1, US 2004/0180186 A1, US 2004/0178544 A1.

### Brief Description of the Drawings

In the following preferred embodiments of the absorbent article according to the invention will be described with reference to the drawings, in which:
- Figure 1: is a schematic top view on a first embodiment of an absorbent article according to the present invention;
- Figure 2: is a schematic top view on a second embodiment of an absorbent article according to the present invention;
- Figure 3: is a schematic top view on a third embodiment of an absorbent article according to the present invention;
- Figure 4: is a schematic top view on a fourth embodiment of the absorbent article according to the present invention;
- Figure 5: is a schematic top view on a fifth embodiment of the absorbent article according to the present invention;
- Figure 5a: is a cross-sectional view along line a-a of Fig. 5;Figure 5b is a cross-sectional view along line b-b of Fig. 5;
- Figure 6a: is a photo of a standard testing fabric which has been engaged with and removed from the absorbent article of the present invention several times;
- Figure 6b: is a photo of a standard testing fabric that has been engaged with and removed from a conventional absorbent article several times; and
- Figures 7-12: are schematic illustrations of preferred methods for manufacturing an absorbent article according to the invention.

### Detailed Description of the Drawings

Absorbent articles, e.g., sanitary napkins, may have quite different shapes, and the specific size and shape of the first and further regions with different engageability generally depends on the shape of the absorbent article so as to optimize both the secure holding of the article in an undergarment and preventing damage to the undergarment.

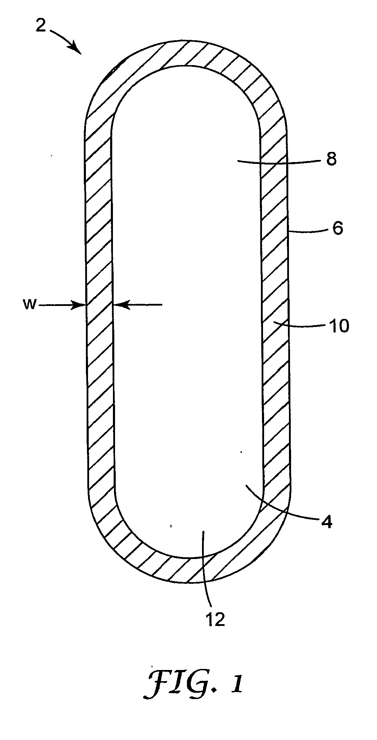

In Figures 1 to 5 several embodiments of typical shapes of the absorbent article 2 of the present invention are schematically illustrated. The absorbent article 2 is adapted for being positioned in an undergarment of a wearer to absorb body exudates. The article 2 comprises a main portion 4 having a liquid-permeable body side topsheet, a liquid-impermeable garment backsheet and being opposite to said topsheet, and a liquid-absorbent core between the body side sheet and the garment side sheet. The article has an outer edge 6 defining the shape of the article and a center area 8. The liquid-impermeable garment side sheet comprises at least two regions with different engagement characteristics (i.e. different engageability). A first region 12 with higher engageability is located at the center area 8 of the article 2, and at least one further region 10 with lower engageability is located between the center area 8 and the outer edge 6. Both regions 10, 12 are provided with mechanical fastening elements.

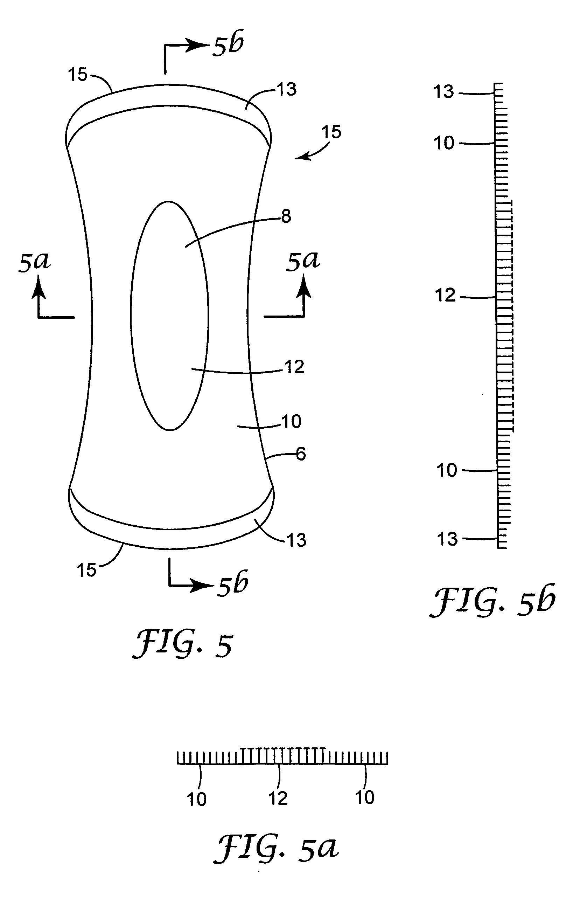

In the embodiments illustrated in Figures 1-4, the article comprises a single further region 10 (i.e. a second region) which preferably extends completely along the edge 6 of the article 2, i.e. around its circumference. However, it is also within the scope of the invention that the article comprises more than one further region 10 (i.e. second, third, etc. regions). As shown in the embodiment of Figure 5, the first region 12 is located in the center area 8 of the article. Two further regions 10, 13 with lower engageability that in the first region 12 are provided. The region 10 (i.e. a second region) is located in the region between the center area 8 and the edge 6 and has a lower engageability than the first region 12. The region 13 (i.e. a third region) is provided only at distinct sections along the transverse edges 15 of the article 2 an may have an even lower engageability than the second region 10.

The width w of the at least one further region 10 around the circumference according to the embodiments of Figures 1-4 may vary or may be constant, and is preferably in the range of between 2 to 20 mm, more preferably between 4 and 10 mm. The first region can be considered to be strip-shaped around the circumference of the articles, said strip having constant or varying width. The width of the third regions 13 of the embodiment according to Figure 5 may be in the same range.

As already mentioned above, the first region 12 is generally located in the center area 8 of the article 2. In other words, the first region 12 is preferably delimited or surrounded by the at least one further region 10. The first region 12 does, however, not necessarily extend to the center, i.e., the first region can also be provide as ring-shaped strip around the center.

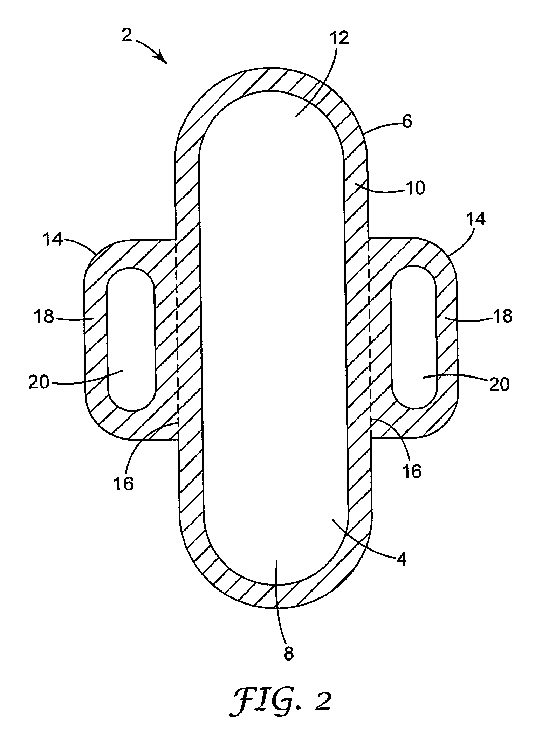

In the embodiment shown in Figure 1, the absorbent article 2 has a generally oval or elliptical shape. In the embodiment according to Figure 2, the absorbent article 2 has generally the same shape as the article shown in Figure 1, but additionally includes flaps 14 at each of its two opposite longitudinal edges. These flaps are adapted to be folded over along fold lines 16 to the opposite side of the undergarment in order to better and more securely affix the article 2 to the undergarment. Preferably, each of the flaps 14 also comprises a first region 20 at its center area with fastening elements of higher engageability and at least one further region 18, preferably along its edge, with lower engageability. Typically, the at least one further region 18 of the flaps 14 comprises the same type of fastening elements as the at least one further region 10 of the main body 4. Furthermore, the first region 20 of the flaps 14 typically comprises the same fastening elements as the first region 12 of the main body 4.

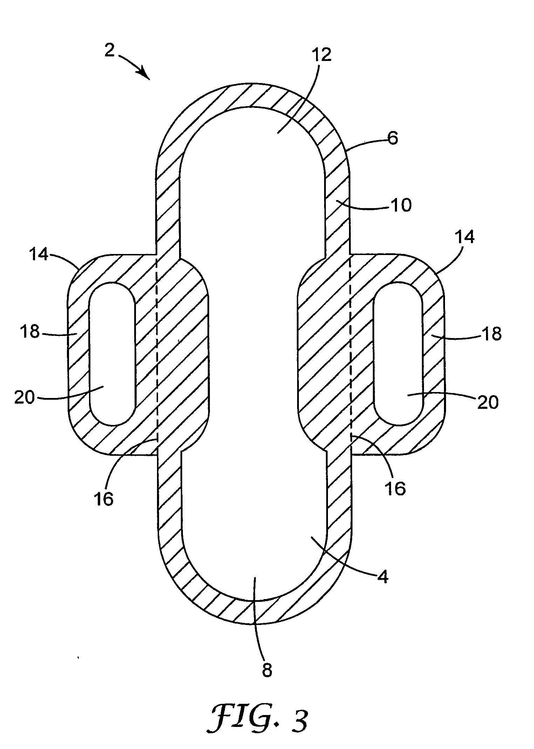

The embodiment shown in Figure 3 generally corresponds to the embodiment of the absorbent article shown in Figure 2, but has a somewhat extended region 10 with lower engageability extending into the main body 4 in the area of the fold lines 16 of the flaps 14.

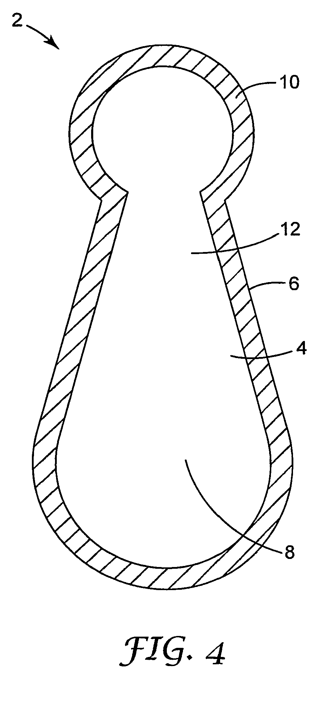

In Figure 4, a generally triangular or keyhole-shaped absorbent article 2 is illustrated. Other shapes of absorbent articles useful in undergarments in order to absorb body exudates are also within the scope of the invention. For example, as shown in Figure 5, the absorbent article may have an hourglass shape.

In order to achieve the different engageability in the first region and the at least one further region several techniques can be used. For example, the fastening elements of the at least one further region 10 may have a lower average height than the fastening elements of the first region 12. According to a specific example, the fastening elements in the at least one further region 10 may have a height of 30 to 400 µm, preferably 75 to 300 µm, more preferably 150 to 250 µm, whereas the fastening elements in the first region 12 may have a height of 50 to 500 µm, preferably 100 to 350 µm, and more preferably 200 to 300 µm.

The engageability of the fastening elements in the various regions may also be varied by using different types of fastening elements in these regions. For example, the fastening elements of the at least one further region 10 may be provided in the form of stems substantially or completely without heads, while the fastening elements of the fist region 12 may be provided in the form of stems with heads, e.g., as hook-type fastening elements, and/or mushroom-type fastening elements. This may be particularly advantageous from a manufacturing point of view since hook-type or mushroom-type fastening elements may be formed from stems by a capping process so that initially the whole fastener, including the first and further regions, can be made from the same precursor material, i.e. a backing with upstanding stems. The different regions are then formed in a final capping process in which only the stems of the first region are capped to form heads, e.g., mushroom-type hooks. Suitable methods for producing mechanical fastening elements in this way are disclosed, e.g., in US-A-5,077,870. Regarding manufacturing techniques as well as hook configurations, sizes and densities further reference is made to WO 94/23610, US-A-5,607,635, US-A-4,894,060, US-A-5,679,302, US-A-5,620,769, US-A-4,794,028, WO 94/04839, US-A-4,984,339 and US-A-5,781,969.

The hook density on the backing is preferably from 30 to 2500 hooks/cm², and more preferably from about 150 to 500 hooks/cm². The stem diameter and/or stem width adjacent the heads of the hooks is preferably from 0.05 to 0.45 mm, and more preferably from about 0.127 to 0.279 mm. The hook head may be circular, elliptical, rectangular, square, hexagonal. For example, a circular disc-like head projects radially past the stem on each side preferably by an average of about 0.013 to 0.254 mm, and more preferably by an average of about 0.025 to 0.127 mm.

To have both good flexibility and strength, the backing of the mushroom-type hook strip preferably may be from 0.025 to 0.512 mm thick, and more preferably from 0.064 to 0.254 mm thick, especially when the hook strip is made of polypropylene or a copolymer of polypropylene and polyethylene. For some uses, a stiffer backing could be used, or the backing can be coated with a layer of pressure sensitive adhesive on its surfaces opposite the hooks by which the backing could be adhered to a substrate so that the backing could then rely on the strength of the substrate to help anchor the hooks.

It is also possible to use both different types of fastening elements and different heights of the fastening elements in the first and the further regions.

Furthermore, different engageability characteristics can be achieved with differently shaped heads or caps of hook-type fastening elements. For example, with rather oval or elliptical heads a higher engageability can be achieved relative to many fabrics, compared with circular heads.

Another possibility is to alter the density of the fastening elements. For example, a lower density of fastening elements per unit area in the at least one further region leads to a lower engageability than with more densely packed fastening elements in the first region.

Another option is to use fastening elements with a smaller stem diameter, i.e. smaller cross-sectional area, in the at least one further region than the fastening elements of the first region. This makes the fastening elements of the at least one further region more flexible than those of the first region which may vary engageability.

It is also possible to combine several or all of these measures in order to adapt the engageability of the fastening elements to the specific requirements in the first and at least one further regions.

It is also within the scope of the invention to provide more than two regions of different engageability. For example, the at least one further region may comprise a second region and a third region, wherein the third region of the absorbent article may be completely free of fastening elements. Such a third region may be provided, for example, at a portion or along the outermost edge of the article, particularly at the transverse (i.e. curved outermost edges of the absorbent article 2) as shown in Figure 5.

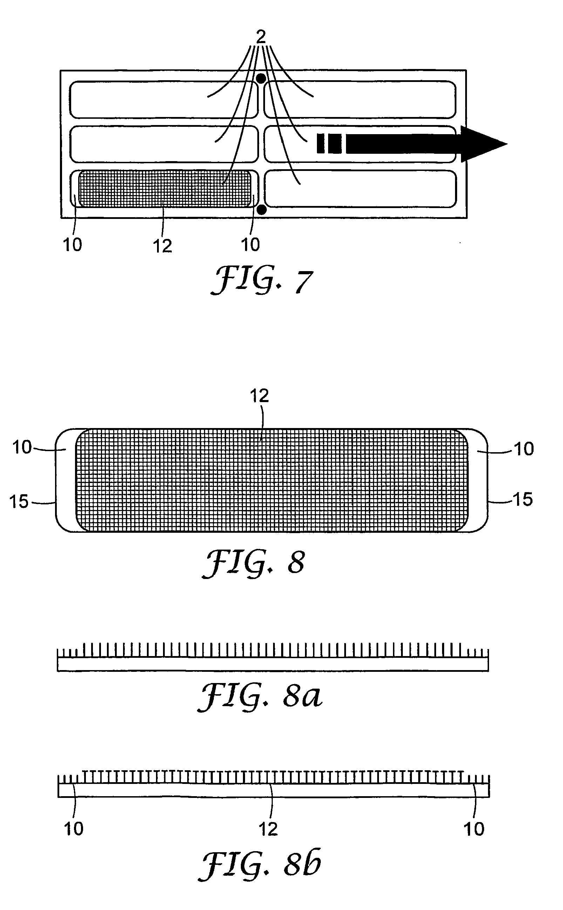

Alternatively, in the embodiments of Figures 1 to 4 the strip-shaped at least one further region 10 may be as a single region. It may also be advantageous to provide two further regions in the upper and lower curved areas of the oval shape of the article, i.e. at the transverse edges 15 as shown for example in the connection with Figures 7 and 8.

Furthermore, the at least one further region 10 and/or the first region 12 of the main body as well as the at least one further region 18 and/or the first region 20 of the flaps 14 may be at least partially provided with an adhesive. The adhesive may be applied in the form of a pattern of varying tackiness. This may also assist in properly positioning the absorbent article in an undergarment.

Generally, the engageability of the at least one further 10 region should be at least 20 %, preferably at least 35 %, and more preferably at least 50 % lower than the engageability of the fastening elements of the first region 12. In particular, it has been found that the peel strength of the fastening elements is primarily responsible for damages to the undergarment upon repeated removal therefrom. It is therefore preferred that the peel strength of the fastening element of the first region 12 is at least two times, preferably at least five times, and more preferably at least 10 times the peel strength of the at least one further region 10. The shear strength of the fastening elements of the at least one further region may be lower, generally the same or higher than the shear strength of the fastening elements of the first region. A higher shear strength of the fastening elements of the at least one further region advantageously contributes in positioning and fixing the absorbent article 2, however, without at the same time causing damage to the undergarment upon removal of the article from the undergarment.

In connection with Figures 7-12 preferred embodiments of manufacturing processes of the absorbent article according to the present invention are schematically illustrated. Generally, the mechanical fastening elements of the absorbent article of the present invention are manufactured by a micro-replication process. This process allows continuous micro-replication of the mechanical fastening elements with a mold, e.g., a drilled belt or drum. As shown in Figure 7, a plurality of absorbent articles 2 can be manufactured on such a belt, wherein the absorbent article is generally oriented with its longitudinal axis in the machine direction which is indicated in Figure 7 by means of an arrow. In Figure 7, only one of the absorbent articles is shown in detail with the first region 12 with a higher engageability and two further regions 10 with lower engageability. The regions with lower engageability are generally provided along the transverse edges 15 of the absorbent article 2. This is shown in Figure 8 in more detail.

As can also be seen in Figure 8, the micro-replication process produces a plurality of upstanding stems, wherein the stems in the first region 12 are higher than the stems in the further regions 10. In a further step, at least the higher stems of the first region 12 are capped by means of a calendaring roll assembly. In this assembly the gap between the stem web and the calendaring roll is such that only the higher stems of the first region 12 get in contact with the roll, whereas the lower stems of the further regions 10 remain uncapped. Thus, in the first region 12 fastening elements with a higher engageability than the fastening elements in the further regions 12 can be produced.

Alternatively, it is also feasible to form a web of upstanding stems with the same height and perform the capping process wih an eccentric or engraved capping roll so that only the stems of the first region 12 will be capped.

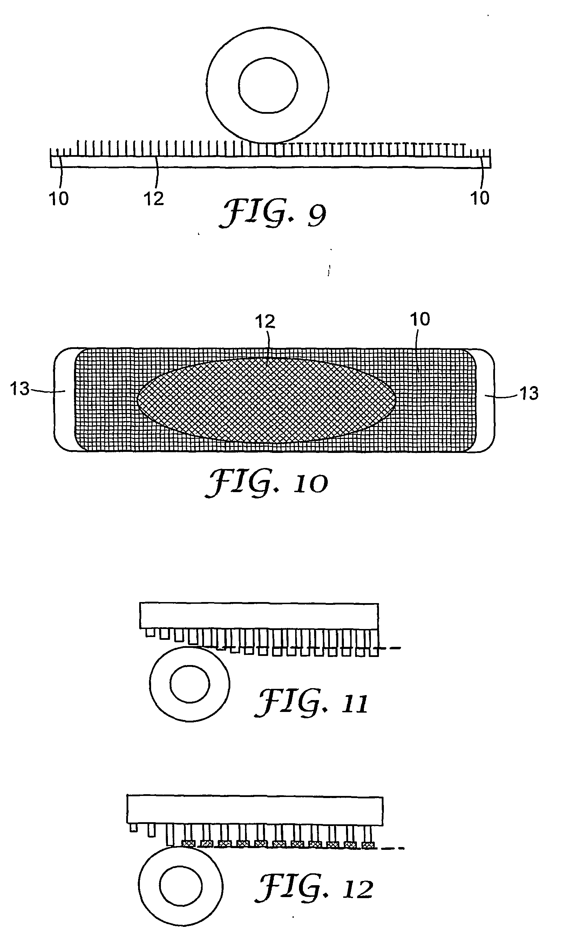

The capping process is schematically illustrated in Figure 9. In this Figure it can be seen, how the calendaring roll produces in the first region 12 mushroom-type fastening elements by capping the taller upstanding stems, while the smaller stems in the further regions 10 are not capped.

In Figure 10, an absorbent article according to the invention is shown that comprises in the center area 8 a first region with fastening elements having a higher engageability and two further regions 10, 13 with fastening elements having lower engageabilites than the first region. In this embodiment, the engageability of the third region 13 is again lower than the engageability of the second region 10.

In Figures 11 and 12, a capping process is shown where stems of increasing height towards the center area 8 of the absorbent article are provided, wherein the dashed line illustrates the border line between stems being capped and the uncapped stem regions.

### Examples

In order to demonstrate the advantageous effects of absorbent articles with lower engageability and higher engageability at appropriate regions of the article several tests have been conducted.

### Tests

### 90° Peel Strength

The 90° peel strength was measured according to ASTM D 5170-91 using a roll down weight of 5000 g. Instead of removing the hook from the fabric just once as described in the above test method this was done twice prior to testing. The tested hook area was 25.4 mm x 15 mm, wherein the 25.4 mm was used as the peel front. The maximum peel force in N/25.4 mm is reported in Table 1 below for several test fabrics.

### Sample preparation:

Hook and fabric material was cut out in a width of 1 inch (25.4 mm). The hook strip was placed onto the fabric and pushed down by a flat steel plate for a period of two seconds. The steel plate was carefully removed and the test specimen was rolled down with a 5 kg roller with 5 cycles. This initial closure was opened by hand and again fastened and opened using the same procedure as described. After the third fastening of the hook into the fabric using 5 cycles of a 5 kg roll down weight the test specimen was positioned into the clamps of the tensile tester being 1 inch (25.4 mm) apart. The testing speed was set to 300 mm/min and the peak force was recorded along a travel distance of the clamps of 50 mm.

### Dynamic Shear Strength

The dynamic shear strength was tested according to ASTM D 5169-91, using a roll down weight of 5000 g. Instead of removing the hook patch from the fabric just once as described in the above test method this was done twice prior to testing. The test area was 25.4 mm x 40 mm. The maximum shear force is reported in Table 2 in N/25.4mm for several test fabrics.

### Sample preparation:

Hook and fabric material was cut out in a width of 1 inch (25.4 mm) having a length of about 4 inch. The hook strip was aligned and placed onto the fabric with an overlap of 40mm. The test specimen was pushed down by a flat steel plate for a period of two seconds. The steel plate was carefully removed and the test specimen was rolled down with a 5 kg roller with 5 cycles. This initial closure was opened by hand with a peel mode and again fastened and opened using the same procedure as described. After the third fastening of the hook into the fabric using 5 cycles of a 5 kg roll down weight the test specimen was positioned into the clamps of the tensile tester being 75 mm apart. The testing speed was set to 300 mm/min and the peak force was recorded.

### Dynamic Friction

The dynamic friction of the first region and the further region was tested on a polypropylene spunbond nonwoven according to DIN 53375.

### Sample preparation:

The fabric (size about 50 mm x 200 mm) was adhered by using a double coated adhesive tape to the sample table. The male component was cut into a pieces of 25.4 x 25.4 mm. One piece of 25.4 x 25.4 mm was adhered with the help of a double coated tape in the geometric center of the slide with a weight of 200 g. The 200 g slide with the male hook patch was positioned onto the fabric so that the male component was in contact with the fabric. The horizontal thread to move the slide is positioned behind a 90° turn into the clamp of the tensile tester. The testing speed was 100 mm/min and the dynamic friction was recorded.

### Test Fabrics

The following test fabrics were used as typical examples for undergarment materials in order to test the 90° peel strength, dynamic shear strength and friction of the fastening elements used in accordance with the invention:
1. Nylon 6.6 (ECE) S/361 standard testing fabric with the designation M.0361, supplied by Testex Prüftextilien, Bad Münstereifel, Germany
2. Polyester fabric (ECE) S/777 standard testing fabric with the designation M.0777, supplied by Testex Prüftextilien, Bad Münstereifel, Germany
3. Cotton standard testing fabric with the designation M.0460, Lot 540, supplied by Testex Prüftextilien, Bad Münstereifel, Germany
4. Typical cotton underwear, bought in a warehouse
5. Typical undergarment made out of 96 % polyamide and 4 % elasthan, bought in a warehouse
6. Polypropylene spunbond with a weight of 40g/m² for friction testing.

### Tested Male Hook Patches

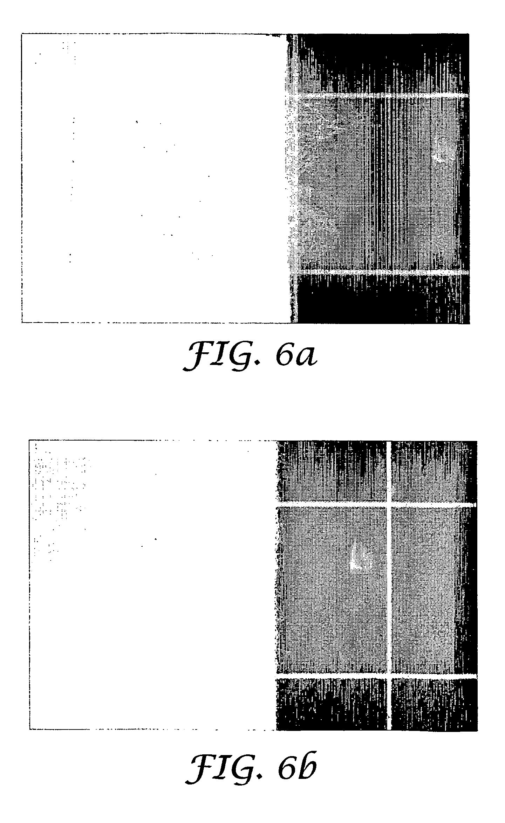

1. A mushroom-type hook web which is commercially available under the trade designation 3M Microreplicated Hook CS-600 from 3M Company, St. Paul, Minnesota, USA. The hook cap (head) geometry is elliptical with the large diameter in cross direction, wherein cross direction means a direction perpendicular to the direction in which the hook itself is manufactured. The density of the hooks is 248 hooks/cm². This type of hook web is described in more detail in US-A-6,000,106, particularly in connection with Figure 9 thereof, and US-A-6,132,660, particularly Figure 6B thereof.
2. A web with stems, i.e., without heads or caps, extending substantially perpendicular from the surface thereof. This material is a precursor web of the CS-600 material mentioned in item 1 above.

**Table 1 - peel strength**

| **Fabric** | **CS-600** | **Stems** |
|---|---|---|
| M.0361 | 0.53 | 0 |
| M.0777 | 0.45 | 0 |
| M.0460 | 0.74 | 0 |
| typical cotton underwear | 0.59 | 0 |
| typical polyamide/elasthan underwear | 2.85 | 0 |

From Table 1 it follows that the peel strength of the CS-600 hook material is considerably higher than that of the stems only, independently from the test fabric used. This means that if stems were used in critical regions of the absorbent article, e.g., the edge region, the fabric of the undergarment would not be damaged because the fasteners would substantially not tear out any fibers. This can be seen, upon a comparison of Figures 6a and 6b. In Figure 6b, the test fabric was M.0361 and the hook material was CS-600 hook material. The hook material and the test fabric were several times engaged and disengaged. This leads to a substantial damage of the fabric, as can be seen in Figure 6b. On the contrary, in Figure 6a the same test fabric was used with a precursor material of the CS-600, i.e. with stems only, wherein after several times of engaging and disengaging the test fabric stays essentially integral without fibers being torn out. Nevertheless, the fastening elements with lower engageability, e.g., the stems tested in these examples, provide additional shear strength and thus contributes to affix the absorbent article reliably on an undergarment.

**Table 2 - shear strength**

| **Fabric** | **CS-600** | **Stems** |
|---|---|---|
| M.0361 | 5.4 | 1.6 |
| M.0777 | 3.1 | 1.6 |
| M.0460 | 5.1 | 1.2 |
| typical cotton underwear | 2.5 | 1.1 |
| typical polyamide/elasthan underwear | 15.2 | 2.6 |

As can be taken from Table 2, the stems as fastening elements of lower engageability provide a lower shear resistance compared to the CS-600 fastening material used in the second region of higher engageability. This means that the stems, while providing substantially no peel resistance with the test fabrics, provides a at least some sheer resistance assisting in the attachment of the absorbent article to the undergarment.

Furthermore, the region with fastening elements of lower engageability also assists in holding the absorbent article on the undergarment due to its higher friction vis-à-vis a region completely free of fastening elements. This can be taken from Table 3 below where the dynamic friction is shown for a precursor web of the CS-600 material, i.e. stems only, on the test fabric M.0460 and on a polypropylene spunbond. For comparison, the same film without stems (here the backside of the same film was used) was tested for dynamic friction with the same test fabrics.

**Table 3 - dynamic friction**

| **Fabric** | **stem side** | **back side of the film with the stems** |
|---|---|---|
| M.0460 | 4.7 | 0.7 |
| polypropylene spunbond | 3.2 | 0.4 |

As can be seen from Table 3, the stem side has much higher dynamic friction forces than the backside of the film, i.e. a film without fastening elements. Thus, the fastening elements of lower engageability still contribute to the fixation of the absorbent article to the undergarment.

## Claims

1. An absorbent article for positioning in an undergarment to absorb body exudates, comprising a main portion having a liquid-permeable body side sheet, a liquid-impermeable garment side sheet opposite to said body side sheet, and a liquid-absorbent core between said body side sheet and said garment side sheet, said article having an outer edge defining the shape of the article and a center area, said liquid-impermeable garment side sheet comprising at least two regions of mechanical fastening elements with different engageability, wherein a first region with higher engageability is located at the center area of the article and at least one further region with lower engageability is located between the center area and the outer edge.

2. The article of claim 1, wherein the fastening elements of the first region have a larger average height than the fastening elements of the at least one further region.

3. The article of claim 1 or 2, wherein the fastening elements of the first region are of a different type than the fastening elements of the at least one further region.

4. The article of any of claims 1 to 3, wherein the fastening elements of the at least one further region are stems without heads and the fastening elements of the first region are hook-type fastening elements.

5. The article of any of claims 1 to 4, wherein the density per unit area of the fastening elements of the at least one further region is lower than the density per unit area of the fastening elements in the first region.

6. The article of any of claims 1 to 5, wherein the fastening elements in the first region and in the at least one further region each have stems, and wherein the stems of the fastening elements of the at least one further region have a smaller cross-sectional area than the stems of the fastening elements of the first region.

7. The article of any of claims 1 to 6, wherein said liquid-impermeable garment side sheet comprises at least two further regions between the center area and the outer edge, wherein the engageability decreases from the center area towards the outer edge of the article.

8. The article of any of claims 1 to 7, wherein at least one of the first region or the at least one further region is at least partially provided with an adhesive.

9. The article of any of claims 1 to 8, wherein a third region is provided adjacent at least portions of the outer edge of the article, said third region being free of fastening elements.

10. The article of any of claims 1 to 9, wherein the main portion has at each of two opposite longitudinal edges flaps that are adapted to be folded over to the opposite side of the undergarment, wherein each of the flaps comprises a first region with fastening elements of higher engageability located at the center area of the flap and at least one further region with fastening elements of lower engageability located between the center area and the edge of the flap.

11. A method of manufacturing an absorbent article for positioning in an undergarment to absorb body exudates, the method comprising the steps of:
a) providing a main portion having a liquid-permeable body side sheet, a liquid-impermeable garment side sheet opposite to said body side sheet, and a liquid-absorbent core between said body side sheet and said garment side sheet;
b) providing on said liquid-impermeable garment side sheet at least two regions of mechanical fastening elements with different engageability, wherein a first region with higher engageability is located at a center area of the article and at least one further region with lower engageability is located between the center area and an outer edge of the article.

12. The method of claim 11, wherein the mechanical fastening elements are provided by a micro-replication process, and wherein micro-replicated stems of the mechanical fastening elements are provided from a master belt, master drum or master screen.

13. The method of claim 12, wherein the master belt, drum or screen is formed as a negative image with a plurality of cavities having differences in at least one of height, diameter, shape, or density in registry to form on a web the plurality of micro-replicated stems corresponding to the plurality of cavities.

14. The method of any of claims 11 to 13, wherein the mechanical fastening elements are provided by first forming a plurality of stems on a substrate web, and then capping the stems in the first region so as to form mushroom-type mechanical fastening elements, wherein at least some of the stems of the at least one further region located generally at the outer edge of the article remain uncapped.

## Patentansprüche

1. Absorbierender Artikel zum Positionieren in einem Unterwäschenstück zum Absorbieren von Körperexsudaten, aufweisend einen Hauptteil mit einem flüssigkeitsdurchlässigen körperseitigen Blatt, einem dem körperseitigen Blatt gegenüberliegenden flüssigkeitsundurchlässigen kleidungsstückseitigen Blatt und einen flüssigkeitsabsorbierenden Kern zwischen dem körperseitigen Blatt und dem kleidungsstückseitigen Blatt, wobei der Artikel einen Außenrand, der die Gestalt des Artikels definiert, und ein Mittelgebiet aufweist, wobei das flüssigkeitsundurchlässige kleidungsstückseitige Blatt mindestens zwei Bereiche an mechanischen Befestigungselementen mit unterschiedlichem Eingreifvermögen aufweist, wobei sich ein erster Bereich mit höherem Eingreifvermögen im Mittelgebiet des Artikels befindet und sich mindestens ein weiterer Bereich mit niedrigerem Eingreifvermögen zwischen dem Mittelgebiet und der Außenkante befindet.

2. Artikel nach Anspruch 1, wobei die Befestigungselemente des ersten Bereichs eine größere Durchschnittsgröße als die Befestigungselemente des mindestens einen weiteren Bereichs aufweisen.

3. Artikel nach Anspruch 1 oder 2, wobei die Befestigungselemente des ersten Bereichs unterschiedlicher Art als die Befestigungselemente des mindestens einen weiteren Bereichs sind.

4. Artikel nach einem der Ansprüche 1 bis 3, wobei die Befestigungselemente des mindestens einen weiteren Bereichs Stiele ohne Köpfe sind und die Befestigungselemente des ersten Bereichs hakenartige Befestigungselemente sind.

5. Artikel nach einem der Ansprüche 1 bis 4, wobei die Dichte pro Flächeneinheit der Befestigungselemente des mindestens einen weiteren Bereichs niedriger ist als die Dichte pro Flächeneinheit der Befestigungselemente des ersten Bereichs.

6. Artikel nach einem der Ansprüche 1 bis 5, wobei die Befestigungselemente in dem ersten Bereich und in dem mindestens einen weiteren Bereich Stiele haben, und wobei die Stiele der Befestigungselemente des mindestens einen weiteren Bereichs eine kleinere Durchschnittsfläche als die Stiele der Befestigungselemente des ersten Bereichs aufweisen.

7. Artikel nach einem der Ansprüche 1 bis 6, wobei das flüssigkeitsundurchlässige kleidungsstückseitige Blatt mindestens zwei weitere Bereiche zwischen dem Mittelgebiet und dem Außenrand aufweist, wobei das Eingreifvermögen vom Mittelgebiet in Richtung des Außenrands des Artikels abnimmt.

8. Artikel nach einem der Ansprüche 1 bis 7, wobei der erste Bereich und/oder der mindestens eine weitere Bereich mindestens teilweise mit einem Klebstoff versehen ist.

9. Artikel nach einem der Ansprüche 1 bis 8, wobei ein dritter Bereich neben mindestens Teilen des Außenrands des Artikels vorgesehen ist, wobei der dritte Bereich frei von Befestigungselementen ist.

10. Artikel nach einem der Ansprüche 1 bis 9, wobei der Hauptteil an jedem zweier sich gegenüberliegenden länglichen Kanten Laschen hat, die dazu ausgelegt sind, auf die gegenüberliegende Seite des Unterwäschenstücks umgefaltet zu werden, wobei jede der Laschen einen ersten Bereich mit Befestigungselementen höheren Eingreifvermögens, der sich im Mittelgebiet der Lasche befindet, und mindestens einen weiteren Bereich mit Befestigungselementen niedrigeren Eingreifvermögens aufweist, der sich zwischen dem Mittelgebiet und der Kante der Lasche befindet.

11. Verfahren zur Herstellung eines absorbierenden Artikels zum Positionieren in einem Unterwäschenstück zum Absorbieren von Körperexsudaten, wobei das Verfahren die folgenden Schritte aufweist:
a) Bereitstellen eines Hauptteils mit einem flüssigkeitsdurchlässigen körperseitigen Blatt, einem dem körperseitigen Blatt gegenüberliegenden flüssigkeitsundurchlässigen kleidungsstückseitigen Blatt, und eines flüssigkeitsabsorbierenden Kerns zwischen dem körperseitigen Blatt und dem kleidungsstückseitigen Blatt;
b) Bereitstellen auf dem flüssigkeitsundurchlässigen kleidungsstückseitigen Blatt mindestens von zwei Bereichen mechanischer Befestigungselemente mit unterschiedlichem Eingreifvermögen, wobei sich ein erster Bereich mit höherem Eingreifvermögen im Mittelgebiet des Artikels befindet und sich mindestens ein weiterer Bereich mit niedrigerem Eingreifvermögen zwischen dem Mittelgebiet und der Außenkante des Artikels befindet.

12. Verfahren nach Anspruch 11, wobei die mechanischen Befestigungselemente durch einen Mikroreplikationsprozess bereitgestellt werden, und wobei mikroreplikierte Stiele der mechanischen Befestigungselemente von einem Hauptband, einer Haupttrommel oder einer Hauptblende bereitgestellt werden.

13. Verfahren nach Anspruch 12, wobei das Hauptband, die Trommel oder die Blende als Negativbild mit mehreren Hohlräumen mit Unterschieden hinsichtlich Höhe und/oder Durchmesser und/oder Gestalt und/oder Dichte im Register ausgebildet ist, um auf einer Bahn die mehreren mikroreplikierten Stiele, entsprechend den mehreren Hohlräumen, zu bilden.

14. Verfahren nach einem der Ansprüche 11 bis 13, wobei die mechanischen Befestigungselemente zuerst durch Bildung mehrerer Stiele auf einer Substratbahn und dann durch Deckelbildung auf den Stielen im ersten Bereich so, dass pilzartige mechanische Befestigungselemente gebildet werden, bereitgestellt werden, wobei mindestens einige der Stiele des mindestens einen weiteren Bereichs, der sich im Allgemeinen an der Außenkante des Artikels befindet, ohne Deckelbildung bleiben.

## Revendications

1. Objet absorbant destiné à être placé dans un sous-vêtement pour absorber les exsudats corporels, doté d'une portion principale qui présente une feuille située du côté du corps et perméable aux liquides, une feuille située du côté du vêtement et imperméable aux liquides, située face à ladite feuille située du côté du corps et une âme centrale qui absorbe les liquides, située entre ladite feuille située du côté du corps et ladite feuille située du côté du vêtement, ledit objet présentant un bord externe qui définit la forme de l'objet et une partie centrale, ladite feuille située du côté du vêtement et imperméable aux liquides comprenant au moins deux zones présentant des éléments de renfort mécanique de résistances différentes, une première zone de résistance plus élevée étant située dans la partie centrale de l'objet et au moins une zone supplémentaire de résistance plus basse étant située entre la surface centrale et le bord externe.

2. Objet selon la revendication 1, dans lequel les éléments de renfort de la première zone ont une hauteur moyenne supérieure à celle des éléments de renfort de la ou des zones supplémentaires.

3. Objet selon la revendication 1 ou 2, dans lequel les éléments de renfort de la première zone sont d'un type différent des éléments de renfort de la ou des zones supplémentaires.

4. Objet selon l'une quelconque des revendications 1 à 3, dans lequel les éléments de renfort de la ou des zones supplémentaires sont des tiges sans tête et les éléments de renfort de la première zone sont des éléments de renfort du type en crochet.

5. Objet selon l'une quelconque des revendications 1 à 4, dans lequel la densité par unité de surface des éléments de renfort de la ou des zones supplémentaires est inférieure à la densité par unité de surface des éléments de renfort dans la première zone.

6. Objet selon l'une quelconque des revendications 1 à 5, dans lequel les éléments de renfort de la première zone et de la ou des zones supplémentaires présentent chacun des tiges et dans lequel les tiges des éléments de renfort de la ou des zones supplémentaires ont une section transversale dont la superficie est inférieure à celle des tiges des éléments de renfort de la première zone.

7. Objet selon l'une quelconque des revendications 1 à 6, dans lequel ladite feuille située du côté du vêtement et imperméable aux liquides comprend au moins deux zones supplémentaires entre la partie centrale et le bord externe, la résistance mécanique diminuant entre la partie centrale et le bord externe de l'objet.

8. Objet selon l'une quelconque des revendications 1 à 7, dans lequel la ou les premières zones ou la ou les zones supplémentaires sont au moins partiellement dotées d'un adhésif.

9. Objet selon l'une quelconque des revendications 1 à 8, dans lequel une troisième zone est prévue au moins à côté de portions du bord externe de l'objet, ladite troisième zone n'étant pas dotée d'éléments de renfort.

10. Objet selon l'une quelconque des revendications 1 à 9, dans lequel la portion principale présente sur chacun des deux bords longitudinaux des rabats qui sont conçus pour être pliés vers le côté opposé du sous-vêtement, chacun des rabats comprenant une première zone avec des éléments de renfort de résistance plus élevée située dans la partie centrale du rabat et au moins une zone supplémentaire dotée d'éléments de renfort de résistance plus basse, cette ou ces zones étant situées entre la partie centrale et le bord du rabat.

11. Procédé de fabrication d'un objet absorbant destiné à être placé dans un sous-vêtement pour absorber des exsudats corporels, le procédé comprenant les étapes qui consistent à :
a) prévoir une partie principale qui présente une feuille située du côté du corps et perméable aux liquides, une feuille située du côté du vêtement et imperméable aux liquides, opposée à ladite feuille située du côté du corps, et une partie centrale qui absorbe les liquides et située entre ladite feuille située du côté du corps et ladite feuille située du côté du vêtement,
b) prévoir sur la feuille latérale située du côté du vêtement et imperméable aux liquides au moins deux zones qui présentent des éléments de renfort mécanique de résistances différentes, une première zone de résistances différentes étant située sur une partie centrale de l'objet et au moins une zone supplémentaire de résistance plus basse étant située entre la partie centrale et un bord externe de l'objet.

12. Procédé selon la revendication 11, dans lequel les éléments de renfort mécanique sont fabriqués par une opération de microréplication et dans lequel les tiges microrépliquées des éléments de renfort mécanique sont obtenues à partir d'une bande-maître, d'un tambour-maître ou d'une toile-maître.

13. Procédé selon la revendication 12, dans lequel la bande-maître, le tambour-maître ou la toile-maître sont formés comme images négatives dotées de plusieurs cavités qui présentent des différences de hauteur, de diamètre, de forme ou de densité de correspondance pour former sur une nappe les différentes tiges microrépliquées qui correspondent aux différentes cavités.

14. Procédé selon l'une quelconque des revendications 11 à 13, dans lequel les éléments de renfort mécanique sont fabriqués en formant d'abord plusieurs tiges sur une nappe de substrat et en chapeautant ensuite les tiges de la première zone de manière à former des éléments de renfort mécanique du type en champignon, au moins certaines des tiges de la ou des zones supplémentaires situées globalement sur le bord externe de l'objet restant non chapeautée.
